# EUROPÄISCHE PATENTANMELDUNG

(11) **EP 1 640 015 A2**
(43) Veröffentlichungstag der Anmeldung: **29.03.2006**
(21) Anmeldenummer: 05020278.7
(22) Anmeldetag: 16.09.2005
(51) Int. Cl.: A61K 36/54, A23L 1/29, A61P 3/04, A61P 3/10

(54) **Arzneimittel und diätetische Lebensmittel, die Zimt und Extrakte aus Zimtrinde enthalten sowie deren Verwendung**

(30) Priorität: 27.09.2004 DE 102004046826
(71) Anmelder: Bioplanta Arzneimittel GmbH, 76275 Ettlingen (DE)
(72) Erfinder: Koch, Egon, Dr., 76229 Karlsruhe (DE); Hauer, Hermann, Dr., 76228 Karlsruhe (DE)
(74) Vertreter: Adam, Holger

(57) **Zusammenfassung**

Die Erfindung betrifft Arzneimittel und diätetische Lebensmittel enthaltend Zimt oder einen Extrakt aus Zimtrinde sowie die Verwendung von Zimt und Extrakten aus Zimtrinde zur Reduktion der Energieaufnahme mit der Nahrung und besonders zur Prophylaxe und Behandlung von Übergewicht, Adipositas und Hyperglykämie sowie von Hyperglykämie-Folgeerkrankungen.

## Beschreibung

Die Erfindung betrifft Arzneimittel und diätetische Lebensmittel enthaltend Zimt oder einen Extrakt aus Zimtrinde sowie die Verwendung von Zimt und Extrakten aus Zimtrinde zur Reduktion der Energieaufnahme mit der Nahrung, und besonders zur Prophylaxe und Behandlung von Übergewicht, Adipositas und Hyperglykämie sowie von Hyperglykämie-Folgeerkrankungen. Fettsucht ist ein weltweites Problem, von dem etwa 100 Millionen Menschen so schwer betroffen sind, dass eine medizinische Intervention erforderlich ist. Besonders gravierend ist die Situation in den westlichen lndustrienationen und in den Schwellenländern. ln den USA sind z. B. zwei Drittel der Bevölkerung übergewichtig, wobei ein Drittel an klinisch relevanter Fettsucht (Adipositas) leidet. Übergewicht und Fettleibigkeit sind direkte oder indirekte Ursache zahlreicher Erkrankungen, wie z. B. Stoffwechselstörungen, Erkrankungen des Bewegungsapparates oder auch neurologischer Leiden. So kann in den Industrienationen unter anderem für die häufigsten Todesursachen (Apoplex, Atherosklerose, Herz-Kreislauf-Erkrankungen, Diabetes, Tumorerkrankungen) eine direkte Beziehung zur Fettleibigkeit hergestellt werden.

Fettsucht (Adipositas) ist definiert als abnormale Akkumulation von Körperfett. Die direkte Bestimmung des Körperfetts duch Unterwasser-Gewichtsbestimmung oder Dual-Energie-Röntgenabsorptionsmessung sind für den Praxisalltag ungeeignet, deshalb werden vorwiegend indirekte Diagnoseverfahren angewendet. Am weitesten verbreitet ist die Berechnung des Körper-Masse-Index (Body-Mass-Index, BMI). Ein BMI zwischen 18,5 bis 25 gilt als normalgewichtig, zwischen 25 und 30 liegt Übergewicht vor und Werte über 30 werden als Fettsucht (Adipositas) definiert.

Neben genetischen Ursachen sind vor allem Umweltfaktoren für die starke Zunahme übergewichtiger Personen verantwortlich. Dazu zählen vor allem geringe körperliche und sportliche Aktivitäten sowie der Konsum von hochenergetischen, fett- und zuckerhaltigen Nahrungsmitteln. Aus diesen Gründen nimmt der Anteil übergewichtiger Personen insbesondere in Schwellenländern, die einen westlichen Lebensstil übernehmen, überproportional stark zu.

Im Vordergrund der Behandlung von Übergewicht und Adipositas steht eine Umstellung des Lebensstils mit erhöhter körperlicher Aktivität und einer Reduktion der Kalorienzufuhr bzw. Umstellung der Ernährungsgewohnheiten. Die Compliance dieser Verfahren ist häufig allerdings gering und für Personen, bei denen auf diese Weise keine Gewichtsreduktion erreicht werden kann, stehen pharmakologische oder chirurgische Methoden zur Verfügung.

Die Pharmakotherapie beruht vor allem auf zentral-aktiven Medikamenten, die den Appetit unterdrücken, und peripher wirkenden Substanzen, die die Fettabsorption reduzieren. Phentermin und Sibutramin sind z. B. zentral wirkende Appetitzügler, die über sympathomimetische Wirkung verfügen (Phentermin) oder die Wiederaufnahme von Noradrenalin und Serotonin (Sibutramin) unterdrücken. Im Gegensatz dazu wirkt Orlistat peripher, indem es die Lipase-Aktivität im Dünndarm hemmt und dadurch die Lipidaufnahme reduziert.

Zimt ist eine der ältesten und begehrtesten Gewürzpflanzen der Welt. Zur Herstellung von Zimt wird die Rinde von Zweigen verschiedener Zimtbaumspezies, die zur Familie der Lorbeergewächse (lat. *Lauraceae)* gehören, verwendet. Die bekanntesten und am häufigsten verwendeten Arten sind der in China beheimatete Cinnamomum aromaticum Nees (Chinazimtbaum, Kassie) und der in Ceylon vorkommende Cinnamomum verum J. S. Presl (Ceylonesischer Zimtbaum, Kaneelbaum). Neben dem sogenannten Stangenzimt, bei dem es sich um die unverarbeitete, getrocknete und eingerollte Rinde handelt, wird Zimt im Handel auch im gemahlenen Zustand angeboten. Als Gewürz wird Zimt vor allem Süßspeisen, Gebäck und Glühwein zugesetzt. Aus den Abfällen wird das Zimtöl gewonnen, das zum Aromatisieren von Likören sowie als Duftstoff in der Parfümindustrie verwendet wird.

Neben der Verwendung als Gewürz wird Zimt auch als Arzneimittel eingesetzt. In der Monographie der Kommission E beim früheren Bundesgesundheitsamt werden für die beiden offizinellen Zimtarten (C. aromaticum und C. verum) folgende pharmakologische Effekte beschrieben: antibakteriell, fungistatisch und motilitätsfördernd. Auf der Grundlage dieser Wirkungen wird die Anwendung bei Appetitlosigkeit, dyspeptischen Beschwerden, Völlegefühl und Blähungen empfohlen. In einer Reihe von Untersuchungen wird außerdem berichtet, daß wäßrige Extrakte aus Zimt die GlucoseAufnahme und die Glykogensynthese in Adipozyten erhöhen (A. Khan et al., (1990) Biol. Trace Elem. Res. 29, 183; C. L. Broadhurst et al. (2000) J. Agric. Food Chem. 48, 849). Diese Effekte beruhen vermutlich auf einer verbesserten Insulin-Rezeptor-Signaltransduktion aufgrund einer Hemmung der Phosphotyrosin-Phophatase 1 B (lmparl-Radosevich J. et al. (1998) Horm. Res. 50, 177-182). Als wirksamkeitsrelevante Inhaltsstoffe wurden ein Hydroxychalcon, dessen Struktur nicht beschrieben wird, und andere Polyphenole postuliert (K. J. Jarvill-Taylor et al. (2001) J. Am. Coll. Nutr. 20, 327; R. A. Anderson et al. (2004) J. Agric. Food Chem. 52, 65).

Der vorliegenden Erfindung liegt die Aufgabe zugrunde, neue Mittel bereitzustellen, die eine Reduktion der Energieaufnahme mit der Nahrung bewirken und somit zur Prophylaxe oder Behandlung von Übergewicht, Adipositas, Hyperglykämie und deren Folgeerkrankungen sowie zur Verminderung des postprandialen Anstiegs der Blutglucose geeignet sind.

Es wurde nun überraschend beobachtet, dass Zimt und Extrakte aus Zimtrinde nicht nur die Phosphotyrosin-Phophatase 1 B inhibieren und die Glucoseaufnahme sowie Glykogensynthese in Muskelzellen erhöhen sondern außerdem auch die Aktivität von Alpha-Amylase hemmen. Alpha-Amylase gehört zu den Verdauungsenzymen und besteht aus zwei verschiedenen lsoenzymen, die beide Stärke und Glycogen abbauen. Als Endprodukt der Amylasespaltung entsteht das Disaccharid Maltose, das durch die Alpha-Glucosidase in Glucose abgebaut wird, die dann über die Darmwand resorbiert werden kann. Alpha-Amylase wird in den Speicheldrüsen und in der Bauchspeicheldrüse gebildet, wobei die Produktion im Pankreas vorherrschend ist und hormonal reguliert wird (z. B. durch Cholecystokinin).

Figur 1 zeigt die Hemmung der Alpha-Amylase aus Schweinepankreas durch einen Extrakt aus der Rinde von Cinnamonum aromaticum (Beispiel 1). Als positive Referenzsubstanz wurde Alpha-Amylase-Inhibitor aus Weizenkeimen geprüft.

Figur 2 zeigt den Einfluß einer oralen Behandlung von Mäusen mit dem Extrakt aus der Rinde von Cinnamonum aromaticum gemäß Beispiel 1 (450 mg/kg) auf den Anstieg des Blutglucosespiegels nach der Verabreichung einer Stärkelösung (2,5 g/10 ml/kg)

Die Alpha-Amylase-hemmenden Eigenschaften von Zimtrindenextrakt wurden mit Hilfe eines In-vitro-Testsystems nachgewiesen (vgl. Beispiel 3), bei dem das Substrat 4,6-Ethyliden-(G7)-p-nitrophenyl-(G1)-α-D-maltoheptaosid (EPS-G7) von Alpha-Amylasen gespalten wird. Diese Spaltprodukte werden dann in einem zweiten Schritt von Alpha-Glucosidase unter Bildung von Glucose und p-Nitrophenol hydrolysiert. Der durch die Bildung von p-Nitrophenol verursachte Extinktionsanstieg wird photometrisch gemessen und ist ein Maß für die Gesamtamylaseaktivität in der Probe.

Durch Hemmung der Alpha-Amylase können die hochmolekularen Kohlenhydrate Amylose und Glycogen nicht zu resorbierbarer Glucose abgebaut werden. Um zu demonstrieren, dass die Alpha-Amylase-hemmenden Eigenschaften von Zimtrinden-Extrakten auch in vivo wirksam sind, wurden tierexperimentelle Untersuchungen durchgeführt (vgl. Beispiel 4).

Aus der Verringerung der Glucose- und damit indirekt der Kalorienaufnahme, ergeben sich günstige Effekte auf die Energiezufuhr und den Blutglucosespiegel, was z. B. bei übergewichtigen Personen oder bei Patienten mit Diabetes mellitus erwünscht ist. Besonders vorteilhaft wirkt sich die Hemmung der Glucosezufuhr in Kombination mit den anderen pharmakologischen Aktivitäten von Zimtrinde (z. B. lnsulin verstärkende Wirkung, antioxidative Eigenschaften) bei Patienten mit Typ II Diabetes aus, die im Gegensatz zu Typ I-Patienten in der Regel nicht an einem Insulinmangel leiden. Typ II-oder Altersdiabetes entsteht infolge einer mangelnden Ansprechbarkeit des Insulinrezeptors an der Zellmembran, was zu einer unzureichenden zellulären Glucoseaufnahme führt. Bei diesen Patienten besteht häufig ein metabolisches Syndrom bei dem neben Störungen des Kohlenhydratstoffwechsel häufig auch pathologische Veränderungen des Fettstoffinrechsels sowie Herz-Kreislauf-Erkrankungen (z. B. Bluthochdruck) auftreten. Antidiabetika (z. B. Sulfonylharnstoffe, Biguanide, Alpha-Glucosidase-Inhibitoren, Thiazolidinedione, Meglitinide, α-linolensäurehaltige Pflanzenöle wie z. B. Perillaöl oder Leinöl), Lipidsenker (z. B. Statine, Fibrate) oder Blutdrucksenker (ACE-Hemmer, Calciumantagonisten, Diuretika, Betablocker) können deshalb vorteilhaft in Kombination mit Zimt und Zimtrindenextrakt angewendet werden.

Gemeinsamer auslösender Faktor der bei Erhöhung des Blutzuckerspiegels (Hyperglykämie) häufig auftretenden Retino- und Makulopathie sowie von Neuropathien sind die durch die Hyperglykämie hervorgerufenen langfristigen biochemischen und zellphysiologischen Veränderungen, die schließlich zu Netzhaut- und Nervenschädigungen führen. An diesen Prozessen sind neben AGEs (advanced glycosylation end products) auch eine vermehrte Metabolisierung von Glucose zu Sorbit und Fruktose, Störungen der zellulären Myoinositolaufnahmen und -synthese, Wechselwirkungen mit der Autoregulation der retinalen Blutzirkulation, die verstärkte Aktivierung von Proteinkinase C und immunologische Prozesse beteiligt. Als besonders nachteilig und eigenständiger Risikofaktor für mikrovaskuläre Komplikationen bei Diabetes wird der postprandiale Anstieg der Blutzuckerspeigel angesehen. Durch Hemmung des Stärkeabbaues wird gerade dieser Parameter besonders günstig beeinflußt.

Positive Wirkungen einer kohlenhydratarmen Diät, umgangssprachlich auch als Atkins-Diät bekannt, wurden wiederholt beschrieben und auch in klinischen Studien (z.B. F. F. Samaha et al., (2003) N. Engl. J. Med. 348, 2074; G. D. Foster et al., (2003) N. Engl. J. Med. 348, 2082) demonstriert. Ein Verzicht auf stärke- und zuckerreiche Nahrungsmittel (z. B. Früchte, Gemüse, Vollkornprodukte) kann aber auch zu einem Mangel von wichtigen anderen Nahrungsbestandteilen (z. B. Vitamine, Mineralstoffe, Spurenelemente etc.) führen. Diese Nachteile werden durch die Verwendung eines Amylase-Inhibitors vermieden, da auf diese Produkte nicht verzichtet werden muß, die erwünschte Reduktion der Energiezufuhr aber gleichwohl erreicht wird. Gegenüber Alpha-Glucosidasehemmern haben Alpha-Amylasehemmer außerdem den Vorteil, daß mit dem Darminhalt deutlich geringere Konzentrationen von Oligo- und Monosacchariden in die unteren Darmabschitte gelangen, wo sie Mikroorganismen als Nährstoffe zur Verfügung stehen. Durch eine Verbesserung der Nährstoffversorgung kann es dort zu einer starken Vermehrung der Keimbesiedlung des Darms mit einer erhöhten Produktion von Stoffwechselprodukten kommen, die Durchfallerkrankungen auslösen können. Gleichwohl kann eine noch effektivere Verminderung der Energiezufuhr aus hochmolekularen Kohlenhydraten durch die Verwendung von Amylase-Inhibitoren in Kombination mit Alpha-Glucosidasehemmern erreicht werden. Außerdem können Kombinationen mit anderen Medikamenten oder Pflanzen bzw. Pflanzenextrakten zur Behandlung von Übergewicht, wie Appetitzügler (z. B. Phentermine, Sibutramin oder Hoodia gordonii) oder Lipasehemmer sinnvoll sein.

Extrakte aus Zimtrinde können nach bekannten Herstellungsverfahren in variabler Zusammensetzung mit Lösungsmitteln wie z. B. Wasser, Methanol, Ethanol, Aceton, etc., und deren Gemische bei Temperaturen von Raumtemp. bis 100 °C unter gelinder bis heftiger Durchmischung innerhalb von 10 Min. bis 24 Std. unter Normaldruck bis zu 200 bar erhalten werden. Zur Anreicherung wirksamkeitsbestimmender Komponenten können weitere Konzentrierungsschritte durchgeführt werden wie z. B. flüssig-flüssig-Verteilung mit z. B. 1-Butanol/Wasser oder Ethylacetat/Wasser, Adsorption-Desorption an lonenaustauscher, LH20, HP20 und andere Harze oder chromatographische Abtrennungen über RP18, Kieselgel, etc.. Falls die Weiterverarbeitung zu Trockenextrakten erwünscht ist, erfolgt diese nach an sich bekannten Verfahren durch Abziehen des Lösungsmittels bei erhöhter Temperatur und / oder reduziertem Druck.

Das erfindungsgemäße Pflanzenmaterial bzw. die daraus hergestellten Extrakte können in Form von Pulvern, Granulaten, Tabletten, Dragees oder Kapseln oder auch als Lösung vorzugsweise oral verabreicht werden.

Zur Herstellung von Tabletten wird der Extrakt mit geeigneten pharmazeutisch verträglichen Hilfstoffen wie z. B. Laktose, Cellulose, Siliciumdioxid, Croscarmellose und Magnesiumstearat gemischt und zu Tabletten gepresst, die gegebenenfalls mit einem geeigneten Überzug z. B. aus Hydroxymethylpropylcellulose, Polyethylenglykol, Farbstoffen (z. B. Titandioxid, Eisenoxid) und Talkum versehen werden.

Das erfindungsgemäße Pflanzenmaterial bzw. die daraus hergestellten Extrakte können auch, ggf. unter Zusatz von Hilfstoffen wie z. B. Stabilisatoren, Füllmittel etc., in Kapseln abgefüllt werden. Die Dosierung erfolgt dabei so, dass pro Tag 0.5 bis 25 g, bevorzugt 1 bis 10 g Zimt bzw. 50 mg bis 10 g, bevorzugt 0.5 bis 5 g Extrakt aus Zimtrinde zugeführt werden.

### Beispiele

### Beispiel 1: Trockenextrakt aus Zimtrinde (Cinnamomum aromaticum)

150 g fein gemahlene Rinde vom Cinnamomum aromaticum werden dreimal mit je 1050 g Ethanol (60 Gew.-%) jeweils 1 h bei 50 °C gerührt. Nach dem Abkühlen auf RT wird filtriert (Seitz 1500). Die vereinigten Filtrate werden im Vakuum bei maximal 50 °C vom Ethanol befreit und gefriergetrocknet: 26,6 g (17,7 %) Trockenextrakt.

### Beispiel 2: Tabletten

Ein Trockenextrakt aus Zimtrinde (Beispiel 1) wird mit Hilfsstoffen gemischt und zu Tabletten verpresst (Tablettenkern = Position 1 - 6). Die Tabletten werden mit einem Überzug aus Hydroxypropylmethylcellulose versehen (Position 7 -10).

| | Bestandteil | mg/Tablette |
|---|---|---|
| 1 | Trockenextrakt aus Zimtrinde | 300,0 |
| 2 | Mikrokristalline Cellulose | 133,0 |
| 3 | Laktose Monohydrat | 58,0 |
| 4 | Croscarmellose | 25,0 |
| 5 | Hochdisperses Siliciumdioxid | 7,0 |
| 6 | Magnesiumstearat | 7,0 |
| 7 | Hydroxypropylmethylcellulose | 21,0 |
| 8 | Polyethylenglycol | 4,5 |
| 9 | Talkum | 1,5 |
| 10 | Titandioxid | 3,0 |

### Beispiel 3: Alpha-Amylase-hemmende Eigenschaften von Zimtrindenextrakt - in vitro Testsystem

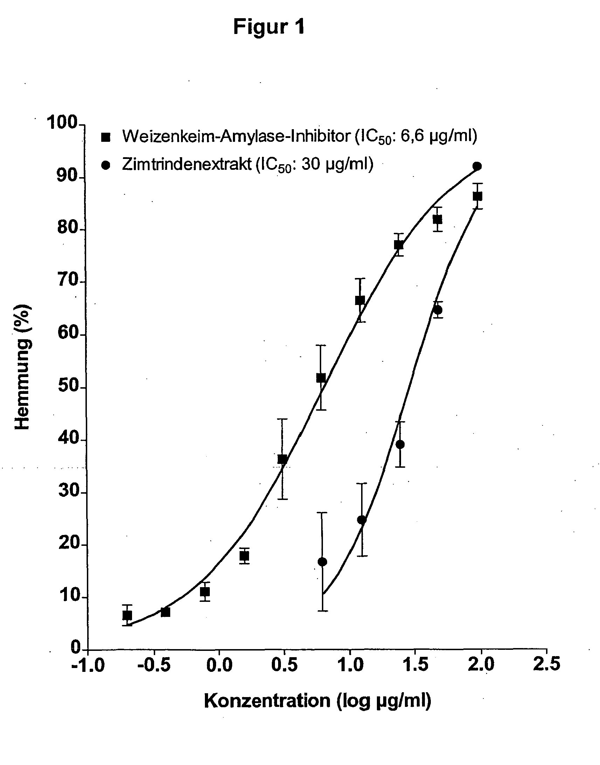

Zur Durchführung des Tests wurden jeweils 37,5 µl Zimtextrakt (gemäß Beispiel 1), Alpha-Amylasehemmer aus Weizenkeimen (Sigma Nr. 1520) als positive Referenzsubstanz oder 2 % DMSO (Lösungsmittelkontrolle) in Phosphatpuffer (100 mM, pH 7,0, 2 g/l bovines Serumalbumin, 0,2 g/l NaN₃) mit 37,5 µl Alpha-Amylase (aus Schweinepankreas, Sigma Nr. 3176, 250 µg/ml Phostphatpuffer) pro Vertiefung in einer F-Form-Mikrotiterplatte gemischt und für 5 min bei Raumtemperatur inkubiert. Anschließend wurde die Enzymreaktion durch Zugabe von 75 µl gebrauchsfertige EPS-G7-Lösung (Rolf Greiner Biochemica, Nr. G10442) gestartet. Die Enzymreaktion wurde kinetisch für 15 min bei 25 °C in einem Mikrotiter-Photometer bei 405 nm gemessen. Die Hemmwirkung von Zimtrindenextrakt und der positiven Referenzsubstanz (Alpha-Amylasehemmer aus Weizenkeimen) wurde im Vergleich zur Lösungsmittelkontrolle (Phosphatpuffer mit 0,5 % DMSO) berechnet. Wie aus der Figur 1 ersichtlich ist, hemmt der Zimtrindenextrakt konzentrationsabhängig die Aktivität von Alpha-Amylase mit einer halbmaximalen Hemmkonzentration von 30 µg/ml.

### Beispiel 4: Alpha-Amylase-hemmende Eigenschaften von Zimtrindenextrakt - in vivo Testsystem

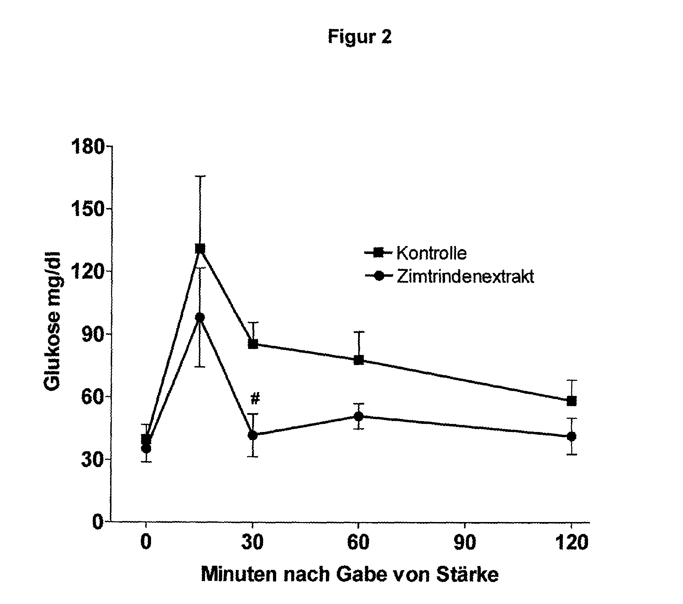

Männlichen NMRI-Mäusen (Janvier, Le Genest, Frankreich; 20-25 g Körpergewicht), denen über Nacht das Futter entzogen wurde, wurden oral mit einem Extrakt (gemäß Beispiel 1) aus der Rinde von C. aromaticum in einer Dosis von 450 mg/kg in 10 ml/kg 0,2 % Agarsuspension behandelt. 30 Minuten später wurde den Mäusen per Schlundsonde eine Stärkelösung (2,5 g/10 ml/kg) verabreicht. Vor Gabe des Zimtrindenextraktes und 15, 30, 45, 60 und 120 min nach Verabreichung der Stärkelösung wurden den Tieren Blutproben aus dem Augenplexus entnommen und der Blutglucose-Spiegel bestimmt (Reflotron Glucose, Roche Diagnostik, Mannheim). Aus den ermittelten Ergebnissen (siehe Figur 2) wird deutlich, daß bei Mäusen, denen der Zimtrindenextrakt verabreicht worden war im Vergleich zu Tieren, die nur das Vehikel (Agarsuspension) erhalten hatten, der Blutglucosespiegel wesentlich weniger stark und für einen kürzeren Zeitraum anstieg.

## Patentansprüche

1. Verwendung von Zimt und Extrakten aus Zimtrinde zur Reduktion der Energieaufnahme mit der Nahrung.

2. Verwendung von Zimt und Extrakten aus Zimtrinde gemäß Anspruch 1, wobei die Reduktion der Energieaufnahme mit der Nahrung durch die Verminderung der Verfügbarkeit von Glucose erreicht wird.

3. Verwendung von Zimt und Extrakten aus Zimtrinde gemäß einem der Ansprüche 1 oder 2, wobei die Reduktion der Energieaufnahme mit der Nahrung zur Prophylaxe oder Behandlung von Übergewicht und/oder Adipositas dient.

4. Verwendung von Zimt und Extrakten aus Zimtrinde gemäß einem der Ansprüche 1 oder 2, wobei die Reduktion der Energieaufnahme mit der Nahrung zur Verminderung des postprandialen Anstieges der Blutglucose führt.

5. Verwendung von Zimt und Extrakten aus Zimtrinde gemäß einem der Ansprüche 1 oder 2, wobei die Reduktion der Energieaufnahme mit der Nahrung zur Prophylaxe oder Behandlung von Hyperglykämie und/oder deren Folgeerkrankungen ausgewählt aus Retino-, Makulo- und Neuropathien dient.

6. Verwendung von Zimt und Extrakten aus Zimtrinde gemäß einem der Ansprüche 4 oder 5 in Kombination mit einem oder mehreren Antidiabetika.

7. Verwendung von Zimt und Extrakten aus Zimtrinde gemäß Anspruch 6, wobei die Antidiabetika aus der Gruppe Sulfonylharnstoffe, Biguanide, Alpha-Glucosidase-Inhibitoren, Thiazolidinedione, Meglitinide, α-linolensäurehaltige Pflanzenöle ausgewählt sind.

8. Verwendung von Zimt und Extrakten aus Zimtrinde gemäß Anspruch 7, wobei das α-linolensäurehaltige Pflanzenöl Perillaöl oder Leinöl ist.

9. Verwendung von Zimt und Extrakten aus Zimtrinde gemäß einem der Ansprüche 1 bis 3 in Kombination mit einem oder mehreren Lipidsenkern und/oder Blutdrucksenkern und/oder Appetitzüglern und/oder Lipasehemmern.

10. Verwendung von Zimt und Extrakten aus Zimtrinde gemäß Anspruch 9, wobei die Lipidsenker ausgewählt sind aus Statinen und Fibraten.

11. Verwendung von Zimt und Extrakten aus Zimtrinde gemäß Anspruch 9, wobei die Blutdrucksenker ausgewählt sind aus ACE-Hemmern, Calciumantagonisten, Diuretika und Betablockern.

12. Verwendung von Zimt und Extrakten aus Zimtrinde gemäß Anspruch 9, wobei die Appetitzügler ausgewählt sind aus der Gruppe Phentermine, Sibutramin oder Hoodia gordonii in unverarbeiteter oder verarbeiteter Form.

13. Arzneimittel oder diätetisches Lebensmittel enthaltend Zimt oder einem Extrakt aus Zimtrinde zur Reduktion der Energieaufnahme mit der Nahrung.

14. Arzneimittel oder diätetisches Lebensmittel gemäß Anspruch 13, wobei die Reduktion der Energieaufnahme mit der Nahrung durch die Verminderung der Verfügbarkeit von Glucose erreicht wird.

15. Arzneimittel oder diätetisches Lebensmittel gemäß einem der Ansprüche 13 oder 14, wobei die Reduktion der Energieaufnahme mit der Nahrung zur Prophylaxe oder Behandlung von Übergewicht und/oder Adipositas dient.

16. Arzneimittel oder diätetisches Lebensmittel gemäß einem der Ansprüche 13 oder 14, wobei die Reduktion der Energieaufnahme mit der Nahrung zur Verminderung des postprandialen Anstieges der Blutglucose führt.

17. Arzneimittel oder diätetisches Lebensmittel gemäß einem der Ansprüche 12 oder 13, wobei die Reduktion der Energieaufnahme mit der Nahrung zur Prophylaxe oder Behandlung von Hyperglykämie und/oder deren Folgeerkrankungen ausgewählt aus Retino-, Makulo- und Neuropathien dient.

18. Arzneimittel oder diätetisches Lebensmittel gemäß einem der Ansprüche 16 oder 17, das zusätzlich ein oder mehrere Antidiabetika enthält.

19. Arzneimittel oder diätetisches Lebensmittel gemäß Anspruch 18, wobei die Antidiabetika aus der Gruppe Sulfonylharnstoffe, Biguanide, Alpha-Glucosidaselnhibitoren, Thiazolidinedione, Meglitinide, α-linolensäurehaltige Pflanzenöle ausgewählt sind.

20. Arzneimittel oder diätetisches Lebensmittel gemäß Anspruch 19, wobei das α-linolensäurehaltige Pflanzenöl Perillaöl oder Leinöl ist.

21. Arzneimittel oder diätetisches Lebensmittel gemäß einem der Ansprüche 13 bis 15, das zusätzlich einen oder mehrere Lipidsenker und/oder Blutdrucksenker und/oder Appetitzügler und/oder Lipasehemmer enthält.

22. Arzneimittel oder diätetisches Lebensmittel gemäß Anspruch 21, wobei die Lipidsenker ausgewählt sind aus Statinen und Fibraten.

23. Arzneimittel oder diätetisches Lebensmittel gemäß Anspruch 21, wobei die Blutdrucksenker ausgewählt sind aus ACE-Hemmern, Caciumantagonisten, Diuretika und Betablockern.

24. Arzneimittel oder diätetisches Lebensmittel gemäß Anspruch 21, wobei die Appetitzügler ausgewählt sind aus der Gruppe Phentermine, Sibutramin oder Hoodia gordonii in unverarbeiteter oder verarbeiteter Form.
